# EUROPEAN PATENT SPECIFICATION

(11) **EP 3 664 798 B1**
(45) Date of publication and mention of the grant of the patent: **29.10.2025**
(21) Application number: 18845286.6
(22) Date of filing: 06.08.2018
(51) Int. Cl.: A61K 31/4025, A61P 35/04

(54) **PHARMACEUTICAL COMPOSITION COMPRISING ELIGLUSTAT**
PHARMAZEUTISCHE ZUSAMMENSETZUNG MIT ELIGLUSTAT
COMPOSITION PHARMACEUTIQUE COMPRENANT DE L'ÉLIGLUSTAT

(30) Priority: 08.08.2017 IN 201721028195
(43) Date of publication of application: 17.06.2020
(60) Divisional application: 25204253.6
(73) Proprietor: Amneal Pharmaceuticals LLC, Bridgewater, NJ 08807 (US)
(72) Inventor: PUROHIT, Parva Yogeshchandra, Ahmedabad Gujarat Ahmedabad 382481 (IN); VASANANI, Paras Rasiklal, Gujarat Ahmedabad 380061 (IN); AGRAWAL, Vikas Maheshbhai, Gujarat Ahmedabad 382415 (IN); SUCHAK, Kishan Pradipbhai, Gujarat Ahmedabad 380019 (IN)
(74) Representative: Haseltine Lake Kempner LLP
(86) International application number: PCT/IB2018/055900
(87) International publication number: WO 2019/030645

(56) References cited:
- WO-A1-2016/038616
- US-A1- 2013 137 743
- US-A1- 2015 038 594
- US-A1- 2015 038 594
- US-A1- 2015 250 856
- US-A1- 2016 331 693
- US-A1- 2016 331 693
- US-A1- 2017 129 869
- US-B2- 7 067 115
- BALWANI ET AL.: "Recommendations for the use of eliglustat in the treatment of adults with Gaucher disease type 1 in the United States", MOLECULAR GENETICS AND METABOLISM, vol. 117, no. 2, 2016, pages 95 - 103, XP029416638

## Description

### Technical Field

The present invention relates to a pharmaceutical composition comprising glucosylceramide synthase inhibitor and at least one pharmaceutically acceptable excipient. Specifically, the present invention relates to a transmucosal pharmaceutical composition comprising eliglustat or a pharmaceutically acceptable salt thereof and at least one pharmaceutically acceptable excipient. Further, the present invention relates to pharmaceutical composition with reduced dose of eliglustat or a pharmaceutically acceptable salt thereof for use in a method for the treatment of one or more lysosomal storage diseases.

### Background Art

Lysosomal storage diseases are a group of rare, inborn, metabolic errors characterized by deficiencies in normal lysosomal function and by intra lysosomal accumulation of undegraded substrates. They are typically characterized by storage of a variety of substrates in multiple tissues, organs and by the variable association of unusual clinical manifestations that are often responsible for physical and neurological handicaps.

Gaucher disease type 1, one of the most common forms of lysosomal storage diseases, is a rare autosomal recessive condition resulting from mutations in the gluco-cerebrosidase (GBA) gene encoding the glucosylceramidase enzyme. In Gaucher disease type 1, the lipid glucosylceramide accumulates in Gaucher cells in organs including the spleen and liver due to insufficient production of the enzyme glucosylceramidase. This leads to clinical manifestations that include enlargement of the spleen and liver, skeletal complications, anaemia and thrombocytopenia.

The current standard of care for Gaucher disease type 1 is enzyme replacement with imiglucerase (recombinant human glucosylceramidase), which can reverse or halt disease progression but is expensive and requires frequent intravenous infusions for the rest of the patient's life. Lifelong intravenous administration, costs and inability of enzyme replacement therapy to reach the CNS and potentially other sites encompass the most important limitations of this treatment modality. Moreover, enzyme replacement therapy is associated with a potential risk of hypersensitivity reactions and, rarely, the development of antibodies to the enzyme that reduce its efficacy.

Substrate reduction therapy aims to decrease the amount of accumulating material by inhibiting its synthesis. Substrate reduction therapy has benefits that could overcome some drawbacks of enzyme replacement therapy. Currently, oral substrate reduction therapy with agents such as miglustat and eliglustat represents an alternative treatment strategy for Gaucher disease type 1.

Eliglustat is a small-molecule oral glucosylceramide analogue for the long-term treatment of Gaucher disease type 1. It is the first oral treatment approved as a first-line use in patients with Gaucher disease type 1 who are CYP2D6 extensive metabolizers (EMs), intermediate metabolizers (IMs), or poor metabolizers (PMs). Eliglustat is a strong inhibitor of glucosylceramide synthase that resembles the ceramide substrate for the enzyme and inhibition of this enzyme reduces the accumulation of glucosylceramide.

Eliglustat is marketed under CERDELGA^{®} brand name which is a hard gelatin capsules containing eliglustat tartrate, with the chemical name N- ((1R,2R)-1-(2,3-dihydrobenzo [b] [1,4] dioxin-6-yl)-1- hydroxy-3- (pyrrolidin-1-yl) propan-2-yl) octanamide (2R,3R)-2,3-dihydroxysuccinate. Its molecular weight is 479.59, and the empirical formula is C23H36N2O4 + ½(C4H6O6) with the following chemical structural Formula - I:

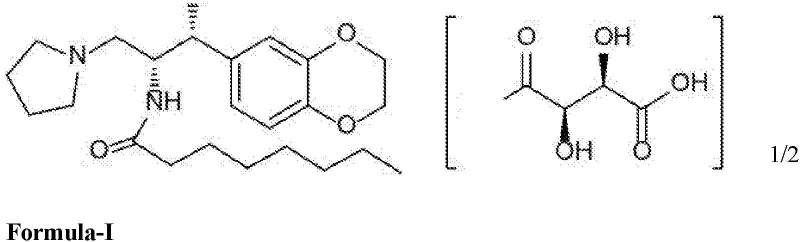

Eliglustat is a BCS class I drug substance characterized by high solubility, high permeability and rapidly absorption. However, the drug has a very low oral bioavailability (<5%) as it undergoes significant first pass metabolism.

As mentioned in CERDELGA^{®} capsule product label, eliglustat is extensively metabolized with a high clearance, mainly by CYP2D6 and to lesser extent CYP3A4 enzymes. At a given dose, the systemic exposure (Cmax and AUC) depends on the CYP2D6 phenotype. In CYP2D6, EMs and IMs, the eliglustat pharmacokinetics is time-dependent and the systemic exposure increases in a more than dose proportional manner. After multiple oral doses of 84 mg twice daily in EMs, eliglustat systemic exposure (AUC0-12) increased up to about 2-fold at steady state compared to after the first dose (AUC0-∞). The pharmacokinetics of eliglustat in CYP2D6 PMs is expected to be linear and time-independent. Compared to EMs, the systemic exposure following 84 mg twice daily at steady state is 7 to 9 fold higher in PMs. The selection of patient with Gaucher disease type 1 is based on their CYP2D6 metabolizer status.

As per the approved product CERDELGA^{®} capsule dosing regimen, it is recommended that patient CYP2D6 genotypes should be established using an FDA-cleared test before starting therapy. The recommended dosage of CERDELGA^{®} capsule is 84 mg twice daily in CYP2D6 EMs and IMs. The recommended dosage in CYP2D6 PMs is 84 mg once daily; appropriate adverse event monitoring is recommended.

As mentioned in European Assessment report for an initial marketing authorization application of CERDELGA^{®} capsule published by Committee for Medicinal Products for Human Use (CHMP), the absolute bioavailability of eliglustat is 4.49% ± 4.13% and the absolute bioavailability was predicted to be approximately 20 times greater for CYP2D6 PMs compared with CYP2D6 EMs.

Lesley J. Scott has also mentioned that, eliglustat has a low oral bioavailability of less than 5 % due to significant first-pass metabolism.

WO2001004108 Al patent application discloses the pharmaceutical composition comprising eliglustat. In particular, the example 3 of WO 2001004108 Al discloses the pharmaceutical composition comprising eliglustat that can be administered orally (e.g., tablets, dragees and capsules), rectally (e.g. suppositories), as well as administration by injection. US2015038594A1 describes orodispersible films comprising a film forming hydrophobic polymer, a disintegrant, a plasticizer and a stabilizer. US2016331693A1 describes all natural, non-toxic sublingual drug delivery systems. US2017129869A1 describes amorphous form of eliglustat hemitartarate. US7067115B2 describes a process and composition for high efficacy teeth whitening. US2013137743A1 describes an amorphous and a crystalline form of Genz 112638 hemitartrate as inhibitor of glucosylceramide synthase. WO2016038616A1 describes NMDA receptor antagonists for treating Gaucher disease. US2015250856A1 describes a pharmaceutical composition for transmucosal delivery and methods for treating diabetes in a subject in need thereof. Balwani et al. provides recommendations for the use of eliglustat in the treatment of adults with Gaucher disease type 1 in the United States in their article in Molecular Genetics and Metabolism, 2016, vol. 117, no. 2, pages 95 - 103.

Hence, there is a long unmet need to develop a suitable pharmaceutical composition which can improve the absolute bioavailability of eliglustat in comparison to marketed CERDELGA^{®} capsule.

Also, there exists a need to improve the currently approved CERDELGA^{®} capsule product dosing regimen for Gaucher disease type 1 which first requires determination of types of patients based on FDA-cleared test for determining CYP2D6 genotype of patients i.e. extensive metabolizers (EMs), intermediate metabolizers (IMs) and poor metabolizers (PMs).

Surprisingly, the inventors of the present invention have addressed the above problems of eliglustat with currently approved product by improving the absolute bioavailability of eliglustat through transmucosal route which circumvents first pass hepatic metabolism. Also, the present invention provides the dose reduction of eliglustat through transmucosal route for Gaucher disease type 1 treatment in comparison to currently approved product.

### Summary of Invention

The present invention relates to a transmucosal pharmaceutical composition as claimed in claim 1. Optional features can be found in claims 2 to 12.

### Description of Embodiments

The present invention relates to a pharmaceutical composition comprising glucosylceramide synthase inhibitor and at least one pharmaceutically acceptable excipient.

Specifically, the present invention relates to a transmucosal pharmaceutical composition of eliglustat or a pharmaceutically acceptable salt thereof and at least one pharmaceutically acceptable excipient for use in a method for the treatment of individuals with one or more lysosomal storage diseases.

In particular, the present invention relates to a sublingual pharmaceutical composition comprising eliglustat or a pharmaceutically acceptable salt thereof. Also described herein is a process of preparing the said pharmaceutical composition.

The present invention addresses major problems associated with the currently approved oral therapy in individuals with Gaucher disease type 1 using eliglustat or a pharmaceutically acceptable salt thereof. Eliglustat is extensively metabolized with a high clearance, mainly by CYP2D6 and to lesser extent CYP3A4 enzymes. At a given dose, the systemic exposure (Cmax and AUC) depends on the CYP2D6 phenotype and it has a very low oral bioavailability of less than 5% through oral route. Also, as mentioned in marketed product CERDELGA^{®} capsule label, no active metabolites have been identified for eliglustat after metabolizing through CYP2D6 enzyme.

The inventors of the present invention have found that the transmucosal administration of eliglustat or a pharmaceutically acceptable salt thereof, specifically sublingual administration offers substantial advantages over oral route by offering improved bioavailability of eliglustat which leads to reduction in administered dose amount in comparison to marketed available CERDELGA^{®} capsule. One of the main advantages of the sublingual administration is the fact that it circumvents exposure of drugs to digestive enzymes in the gastrointestinal tract and avoids the first pass effect from hepatic enzymes immediately upon absorption. The direct access to blood circulation in addition to the avoidance of any metabolism of the drug results in achieving quickly the maximum levels of the active ingredient in the plasma. Thus, a faster onset of pharmacological effects of the drug in patients is achieved in comparison to conventional oral delivery where the composition is swallowed and also dose reduction is possible. Also, the present invention improves the currently approved CERDELGA^{®} capsule product dosing regimen for Gaucher disease type 1 which first requires determination of types of patients based on FDA-cleared test for determining CYP2D6 genotype of patients i.e. extensive metabolizers (EMs), intermediate metabolizers (IMs) and poor metabolizers (PMs).

The term "eliglustat" encompasses the compound eliglustat or its pharmaceutically acceptable salts or esters or pro drugs thereof or the active metabolites of eliglustat or any of their polymorphs, solvates, hydrates, and combinations thereof such as hydrated salts of eliglustat.

The term "transmucosal" refers to a drug administration route through mucosal lining including the mucosal linings of the nasal, rectal, vaginal, ocular, or oral cavity. Specifically in present invention, the term "transmucosal" refers to a drug administration route through mucosal linings of the oral cavity i.e. sublingual and buccal. The inventors of present invention have emphasized specifically on administration of pharmaceutical composition through sublingual route throughout specification, however, administration of pharmaceutical composition is not limited by sublingual route, it may be administered through other transmucosal routes i.e. buccal, gingival or a like thereof.

As used herein, the term "therapeutically effective amount" refers to the amount of eliglustat that is capable of achieving a therapeutic effect in individuals in need of treatment of one or more lysosomal storage disease.

A "pharmaceutically acceptable salt" means any non-toxic salt or salt of an ester of a compound which upon administration into a patient is capable of providing, either directly or indirectly, a compound of this invention. Suitable examples of pharmaceutically acceptable salts include a tartrate, succinate, maleate, fumarate, malate, hydrochloride, hydrobromide, sulfate etc.

The term "about" refers to any value which lies within the defined range by present inventors from a variation of up to ±10% of the claimed value.

The term "stable" means a drug substance and/or pharmaceutical composition for pharmaceutical use which remains stable as per ICH guidelines and/or pharmacopoeia guidelines.

The term "shelf life" refers to storage stability time period during which drug product and/or drug substance expected to remain stable within defined specification as per ICH guidelines and/or pharmacopoeia guidelines.

In first aspect, the present invention relates to a transmucosal pharmaceutical composition comprising eliglustat tartrate and a one or more pharmaceutically acceptable excipients which provides enhanced bioavailability and reduces dose of eliglustat in comparison to marketed CERDELGA^{®} capsule.

In second aspect, the present invention relates to a sublingual pharmaceutical composition comprising eliglustat tartrate and a one or more pharmaceutically acceptable excipients.

In third aspect, the present invention relates to a buccal pharmaceutical composition comprising eliglustat tartrate and a one or more pharmaceutically acceptable excipients.

The transmucosal pharmaceutical composition of the present invention may present in the form of tablets, granules, pellets, films, lozenges, wafers, spray, drops, effervescent sublingual or buccal tablets, pastilles or a like thereof.

Further, the pharmaceutical composition of the present invention disintegrates in the oral cavity of a patients about 60 seconds or less, about 50 seconds or less, about 40 seconds or less, about 30 seconds or less or about 20 seconds or less or about 10 seconds or less.

Preferred pharmaceutical compositions are solid pharmaceutical compositions which rapidly disintegrate in the mouth of a subject, upon insertion into the buccal pouch or between the gum and cheek, or upon placement under the tongue without leaving an unpleasant taste in mouth. Rapid disintegration means that the pharmaceutical composition is disintegrated within 60 seconds in water at 37° C., and preferably within 30 seconds.

Further, the pharmaceutical composition of the present invention dissolves or disperses rapidly in the oral cavity after administration.

According to one embodiment of the present invention, there is provided a sublingual pharmaceutical composition of eliglustat or a pharmaceutically acceptable salt thereof, wherein the eliglustat or a pharmaceutically acceptable salt thereof is present in amount from about 1 mg to about 30 mg, wherein the transmucosal pharmaceutical composition is for use in a method comprising administration through the mucosal lining of sublingual or buccal in oral cavity. The eliglustat or a pharmaceutically acceptable salt thereof may be present in an amount from about 1% to about 80% by weight of composition, preferably from about 5% to 40% by weight of composition.

According to another embodiment of the present invention, there is provided a sublingual pharmaceutical composition of eliglustat or a pharmaceutically acceptable salt thereof and at least one pharmaceutically acceptable excipient selected from diluents, binders, disintegrants, super-disintegrants, lubricants, glidants, sweetening agents, flavoring agents, taste-masking agents, coloring agents, film-forming agents, coating agents and mixtures thereof.

The diluents used in the pharmaceutical composition of the present invention are selected from the group consisting of maize starch, potato starch, rice starch, wheat starch, pregelatinized starch, lactose monohydrate, lactose anhydrous, microcrystalline cellulose, silicified microcrystalline cellulose, confectioner's sugar, partially hy- drolysed gelatin and like thereof. Other useful diluents include, but are not limited to, sugar alcohols such as mannitol, sorbitol, and xylitol, calcium carbonate, magnesium carbonate, dibasic calcium phosphate, and tribasic calcium phosphate. The diluents may present in an amount from about 5% to about 80% by weight of composition, preferably from about 30% to about 80% by weight of composition.

The binders used in the pharmaceutical composition of the present invention are selected from the group consisting of a starches, natural and synthetic gums, cellulose derivatives, gelatin, povidone, copovidone, polyethylene glycol, waxes, sodium alginate, alcohols, water, and the like thereof. The binders may present in an amount from about 01% to 20% by weight of composition, preferably from about 3% to 15% by weight of composition, and more preferably from about 1% to about 5% by weight of composition.

The disintegrants and super-disintegrants used in the pharmaceutical composition of the present invention are selected from the group consisting of cross-linked polymer such as crospovidone (crosslinked PVP), modified starches such as sodium starch glycolate, cross-linked cellulose such as crosslinked sodium carboxymethyl cellulose (croscarmellose sodium), low substituted hydroxypropyl cellulose, cross-linked alginic acid, natural polymer such as soy polysaccharides, ion-exchange resins, calcium silicate and mixtures thereof. According to the present invention, preferable disintegrants are crospovidone, croscarmellose sodium and sodium starch glycolate. The disintegrants may present in an amount from about 1% to 30% by weight of composition, preferably from about 1% to 25% by weight of composition, and more preferably employed in an amount in a range of from about 5% to 20% by weight of composition.

The lubricants used in the pharmaceutical composition of the present invention are selected from the group consisting of a calcium stearate, Glyceryl monostearate, Glyceryl palmitostearate, hydrogenated castor oil, hydrogenated vegetable oil, light mineral oil, magnesium stearate, mineral oil, polyethylene glycol, stearic acid, , zinc stearate, and sodium stearyl fumarate and a combination thereof. A preferred lubricant is magnesium stearate and sodium stearyl fumarate and may present in amount from about 0% to about 10% by weight of composition, preferably from about 0.5% to about 5% by weight of composition and more preferably from about 1% to about 2% by weight of composition.

The glidants used in the pharmaceutical composition of the present invention are selected from the group consisting of a starch, talc, lactose, stearates, dibasic calcium phosphate, magnesium carbonate, magnesium oxide, calcium silicate, and colloidal silicon dioxide and the like thereof. The Glidants may present in an amount from about 0% to about 10% by weight of composition, preferably from about 0.5% to about 5% weight of composition more preferably from about 1% to about 2% by weight of com- position.

The sweetening agents used in the pharmaceutical composition of the present invention are selected from the group consisting of a alitame, acesulfame potassium, aspartame, D-tryptophan, dextrose, erythritol, fructose, galactose, glycerol, gly-cyrrhizin, glucose, isomalt, xylitol, xylose, lactitol, lactose, levulose, maltitol, maltodextrin, maltol, maltose, corn syrup, neohesperidin dihydrochalcone, neotame, sodium saccharin, siclamate, sorbitol, sucralose, sucrose, tagatose, taumatin, trehalose, and the like thereof. The sweetening agents may present in an amount of about 10% or less by weight of composition, preferably about 1% or less by weight of composition.

The flavoring agents used in the pharmaceutical composition of the present invention are selected from the group consisting of a natural flavoring oils, anethole, acetic acid, ascorbic acid, phosphoric acid, fumaric acid, lactic acid, lemon, linalool, malic acid, menthol, eucalyptol, orange, cinnamone, tartaric acid, thymol, vanilla, strawberry, cherry Flavor (spray dried naturaltype), chocolate aroma or peppermint aroma and the like thereof. The flavoring agents may present in an amount of about 1% or less by weight of composition, preferably less than about 0.80% or more preferably less than about 0.70% by weight of composition.

The coloring agents used in the pharmaceutical composition of the present invention are selected from the group consisting of natural and/or artificial materials such as FD&C coloring agents, natural juice concentrates, pigments such as titanium oxide, iron oxides, silicon dioxide, and zinc oxide, combinations thereof, and the like. The coloring agents may present in an amount of about 1% or less by weight of composition, preferably less than about 0.80% or more preferably less than about 0.70% by weight of composition.

The film-forming or coating agents used in the pharmaceutical composition of the present invention are selected from the group consisting of cellulose derivatives such as methylcellulose, hydroxymethyl cellulose, hydroxyethyl cellulose, hydroxypropyl cellulose, hydroxymethyl ethylcellulose, hydroxypropyl methylcellulose, sodium carboxymethyl cellulose, and ethyl cellulose; waxes; fat substances; or mixtures thereof. Alternatively, commercially available coating compositions comprising film forming polymers marketed under various trade names, such as Opadry^{®}, may be used for coating.

Examples of solvents used for preparing the coating solution as well as granulating solution are selected from methyl alcohol, ethyl alcohol, isopropyl alcohol, n-butyl alcohol, acetone, acetonitrile, chloroform, methylene chloride, water, or mixtures thereof.

In another embodiment, the object of the present invention is achieved by employing an effective amount of a pH dependent excipient as a taste-masking agent that is insoluble in acidic environment and soluble in neutral or alkaline conditions, in order to reduce irritation with oral mucosa, such as tongue and mouth mucosa.

The taste-masking agents used in the pharmaceutical composition of the present invention are selected from the group consisting of ion-exchange resins, Opadry^{®} AMB TAN, polymethacrylates (especially Eudragit^{®} L100), sodium starch glycolate, carbopol polymers, PEG-5M, sodium acetate, ethylcellulose, betacyclodextrin, polyvinyl acetate dispersion, trehalose, vinylacetate, polystyrene and cellulose acetate butyrate. Preferably, anionic copolymers based on methacrylic acid and methyl methacrylates such as Eudragit^{®} L100 are used in present invention.

The pharmaceutical composition of the present invention can be obtained by a known conventional methods like dry granulation, wet granulation, direct compression, roller compaction, fluidized bed granulation, solid-dispersion, rapid mixture granulation, solvent evaporation, hot-melt extrusion, freeze-drying, lyophilisation, or a like thereof.

The pharmaceutical composition for transmucosal administration of the present invention is characterized by physicochemical properties to evaluate adequate release rate of the active ingredient from composition and also to achieve storage stability e.g. disintegration time, dissolution rate, hardness, friability and stability.

Also described herein isa formulation comprising a solid dispersion or intimate mixture of a drug and a polymer, wherein said polymers are selected from the group consisting of hypromellose, copovidone, povidone, methyl cellulose, hydroxyethyl cellulose, hydroxypropyl cellulose, ethyl- cellulose, pyroxylin, polyethylene oxide, polyvinyl alcohol, polyethylene glycol, and polyvinyl caprolactam-polyvinyl acetate-polyethylene glycol, polysaccharides, polypeptides, and methacrylic acid copolymers, ethyl acrylic acid copolymers. A preferred polymer is acrylic products such as poly(meth)acrylate (EUDRAGIT^{™}) copolymers are available in various physical forms, for example, EUDRAGIT EPO being a powder form of EUDRAGIT E 100. The polymers may present in an amount from about 1 to about 60% by weight of composition, preferably from about 5 to about 45% by weight of composition and is more preferably employed in an amount in a range of from about 10 to about 30 % by weight of composition.

Optionally, the pharmaceutical composition of the present invention may further be film-coated using techniques well known in the art such as spray coating in a conventional coating pan or a fluidized bed processor or dip coating. Alternatively, coating may also be performed using the hot melt technique. The film coat comprises film-forming polymers, a one or more pharmaceutically acceptable excipients and pharmaceutically acceptable solvents.

Also described herein is a sublingual pharmaceutical composition comprising from about 1% to about 40 % w/w of eliglustat or a pharmaceutically acceptable salt thereof, from about 10% to about 80% w/w of diluent, from about 01 to about 20% w/w of binder, from about 0.5% to about 20% w/w of disintegrant including any super-disintegrant, from about 0% to about 5% w/w of lubricant, from about 0% to about 5% w/w of glidant, from about 10% w/w or less of sweetening agent, from about 10% w/w or less of flavoring agent, from about 0% to about 20% w/w of taste-masking agents, from about 5% w/w or less of coloring agent and optionally from about 1% to about 10% w/w of film coating substance.

According to another embodiment of the present invention, there is provided a sublingual pharmaceutical composition comprising from about 5% to about 30% w/w of eliglustat tartrate, from about 30% to about 70% w/w of diluent, from about 2% to about 8% w/w of binder, from about 5% to about 20% w/w of disintegrant, from about 0% to about 3% w/w of lubricant, from about 0% to about 3% w/w of glidant, from about 5% w/w or less of sweetening agent, from about 5% w/w or less of flavoring agent, from about 0% to about 10% w/w of taste-masking agents, from about 3% w/w or less of coloring agent and optionally from about 1% to about 5% w/w of film coating substance.

Also described herein is a process for the preparation of a pharma- ceutical composition of the present invention, wherein the process comprises the steps of-Preparing a blend of eliglustat or a pharmaceutically acceptable salt thereof with fillers/diluents; mixing said blend with one or more pharmaceutically acceptable excipients specifically super-disintegrants; subsequently lubricating the blend; and at last directly compressing the lubricated blend into tablets and optionally coating the said composition.

Also described herein is a process for the preparation of a pharma- ceutical composition of the present invention, wherein the process comprises the steps of-Preparing a dry mixture of eliglustat or a pharmaceutically acceptable salt thereof and fillers/diluents; blending said dry mixture with one or more pharmaceutical excipients specifically super-disintegrants; further solvent is slowly sprayed onto the powder for the granulation purpose; blending the obtained granules with extra-granular excipients and lubricating the blend; at last compressing the obtained lubricated blend to form a tablet and optionally coating the said composition.

Also described herein is a process for the preparation of a pharmaceutical composition of the present invention, wherein the process comprises the steps of- blending eliglustat or a pharmaceutically acceptable salt thereof, polymers and one or more pharmaceutical excipients in high shear mixer to obtain granules; loading the granules obtained in into a hot melt extruder to form a pellets in the form of extrudates; milling the extrudates and adding one or more fillers, disintegrants, and lubricants; compressing the granules into tablets and optionally coating the said composition.

Also described herein is a process for the preparation of a pharma- ceutical composition of the present invention, wherein the process comprises the steps of-Mixing the eliglustat or a pharmaceutically acceptable salt thereof in a matrix consisting of a polymeric structure former, e.g. partially hydrolyzed gelatin, and a saccharide, typically mannitol, dissolved in water and formulated into a liquid solution or suspension; the liquid is precisely filled into pre-formed blisters and passed through a specially designed cryogenic freezing process to control the ultimate size of the ice crystals; the frozen units are then transferred to freeze dryers for the lyophilisation process; the blisters containing the dried units are then sealed via a heat-seal process to protect the product from varying environmental conditions and ensure long-term stability.

In another embodiment of the present invention, there is provided a sublingual film composition comprising therapeutically effective amount of eliglustat or a pharmaceutically acceptable salt and a one or more pharmaceutically acceptable excipients.

In another embodiment, the present invention relates to a sublingual film composition comprising eliglustat or a pharmaceutically acceptable salt thereof and a one or more pharmaceutically acceptable excipients which provides enhanced bioavailability and reduces dose of eliglustat in comparison to marketed CERDELGA^{®} capsule.

Also described herein is a sublingual film composition of eliglustat or a pharmaceutically acceptable salt thereof, wherein the eliglustat or a pharmaceutically acceptable salt thereof is present in amount from about 1 mg to about 100 mg. Preferably, from about 5 mg to about 50 mg, and more preferably from 10 mg to 30 mg of eliglustat or a pharmaceutically acceptable salt thereof. The eliglustat or a pharmaceutically acceptable salt thereof is present in an amount from about 1% to about 80% by weight of composition, preferably from about 1% to 20% by weight of composition.

The sublingual film composition described herein dissolves rapidly in the oral cavity after administration.

The polymer included in the films may be water-soluble, water-swellable, water-insoluble, or a combination of one or more either water-soluble, water-swellable or water-insoluble polymers. The polymer may include cellulose or a cellulose derivative. Specific examples of useful water-soluble polymers include, but are not limited to, polyethylene oxide, pullulan, hydroxypropylmethyl cellulose, hydroxyethyl cellulose, hydroxypropyl cellulose, polyvinyl pyrrolidone, carboxymethyl cellulose, polyvinyl alcohol, sodium alginate, polyethylene glycol, xanthan gum, tragancanth gum, guar gum, acacia gum, arabic gum, polyacrylic acid, methylmethacrylate copolymer, carboxyvinyl copolymers, starch, gelatin, and combinations thereof. Specific examples of useful water-insoluble polymers include, but are not limited to, ethyl cellulose, hydroxypropyl ethyl cellulose, cellulose acetate phthalate, hydroxypropyl methyl cellulose phthalate and combinations thereof. The polymers may present in an amount from about 20% to 80% by weight of composition, preferably from about 25% to 70% by weight of composition, and more preferably from about 30% to about 60% by weight of composition.

Other polymers useful for incorporation into the films include biodegradable polymers, copolymers, block polymers and combinations thereof. It is understood that the term "biodegradable" is intended to include materials that chemically degrade, as opposed to materials that physically break apart (i.e., bioerodable materials). Among the known useful polymers or polymer classes which meet the above criteria are: poly(glycolic acid) (PGA), poly(lactic acid) (PLA), polydioxanes, polyoxalates, poly(α-esters), polyanhydrides, polyacetates, polycaprolactones, poly(orthoesters), polyamino acids, polyaminocarbonates, polyurethanes, polycarbonates, polyamides, poly(alkyl cyanoacrylates), and mixtures and copolymers thereof. Additional useful polymers include, stereopolymers of L- and D-lactic acid, copolymers of bis(p-carboxyphenoxy)propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of polyurethane and poly(lactic acid), copolymers of α-amino acids, copolymers of α-amino acids and caproic acid, copolymers of α-benzyl glutamate and polyethylene glycol, copolymers of succinate and poly(glycols), polyphosphazene, polyhydroxy-alkanoates and mixtures thereof. Binary and ternary systems are contemplated.

A variety of components and fillers also may be added to the films. These may include, without limitation: surfactants; plasticizers; polyalcohols; anti-foaming agents, such as silicone-containing compounds, which promote a smoother film surface by releasing oxygen from the film; thermo-setting gels such as pectin, carageenan, and gelatin, which help in maintaining the dispersion of components; inclusion compounds, such as cyclodextrins and caged molecules; coloring agents; taste-masking agents, saliva stimulating agents and flavoring agents. In some embodiments, more than one active component may be included in the film.

Useful additives include, for example, gelatin, vegetable proteins such as sunflower protein, soybean proteins, cotton seed proteins, peanut proteins, grape seed proteins, whey proteins, whey protein isolates, blood proteins, egg proteins, acrylated proteins, water-soluble polysaccharides such as alginates, carrageenans, guar gum, agar-agar, xanthan gum, gellan gum, gum arabic and related gums (gum ghatti, gum karaya, gum tragancanth), pectin, water-soluble derivatives of cellulose: alkylcelluloses hydrox-yalkylcelluloses and hydroxyalkylalkylcelluloses, such as methylcelulose, hydrox-ymethylcellulose, hydroxyethylcellulose, hydroxypropylcellulose, hydroxyethyl-methylcellulose, hydroxypropylmethylcellulose, hydroxybutylmethylcellulose, cellulose esters and hydroxyalkylcellulose esters such as cellulose acetate phthalate (CAP), hydroxypropylmethylcellulose (HPMC); carboxyalkylcelluloses, carboxyalky-lalkylcelluloses, carboxyalkylcellulose esters such as carboxymethylcellulose and their alkali metal salts; water-soluble synthetic polymers such as polyacrylic acids and polyacrylic acid esters, polymethacrylic acids and polymethacrylic acid esters, polyvinyl acetates, polyvinylalcohols, polyvinylacetatephthalates (PVAP), polyvinylpyrrolidone (PVP), PVY/vinyl acetate copolymer, and polycrotonic acids; also suitable are phthalated gelatin, gelatin succinate, crosslinked gelatin, shellac, water-soluble chemical derivatives of starch, cationically modified acrylates and methacrylates possessing, for example, a tertiary or quaternary amino group, such as the diethylaminoethyl group, which may be quaternized if desired; and other similar polymers.

The plasticizers used in the pharmaceutical composition of the present invention are selected from the group consisting of polyalkylene oxides, such as polyethylene glycols, polypropylene glycols, polyethylene-propylene glycols, organic plasticizers with low molecular weights, such as glycerol, glycerol monoacetate, diacetate or triacetate, triacetin, polysorbate, cetyl alcohol, propylene glycol, sorbitol, sodium diethylsulfosuccinate, triethyl citrate, tributyl citrate, and the like. The plasticizers may present in amount from about 0.5% to about 40%, preferably from about 5% to about 20% based on the weight of composition.

The saliva stimulating agents used in the pharmaceutical composition of the present invention are selected from the group consisting of citric acid, malic acid, lactic acid, ascorbic acid or like thereof.

The film compositions further desirably contains a buffer so as to control the local pH of the film composition. Any desired level of buffer may be incorporated into the film composition so as to provide the desired local pH level. The buffer is preferably provided in an amount sufficient to control the release of active ingredient from the film and/or the absorption into the body. The film composition preferably has a local pH of about 5 to about 7.

Also described herein is a
sublingual film composition comprising from about 1% to about 30 % w/w of eliglustat tartrate , from about 10% to about 60% w/w of polymer, from about 5 to about 40% w/w of plasticizer, from about 0% to about 20% w/w of saliva stimulating agent, from about 0% to about 10% w/w of sweetening agent, from about 0% to 0.1% w/w of flavoring agent, from about 0% to about 20% w/w of taste-masking agent and optionally from about 5% w/w or less of coloring agent.

The sublingual pharmaceutical film of the present invention can be prepared by known conventional methods like solvent casting method, semisolid casting method, hot melt extrusion, solid dispersion extrusion and rolling method.

The sublingual pharmaceutical film of the present invention is optimised to achieve desired characteristics like film thickness, weight variation, pH, folding endurance, tensile strength, moisture content, in vitro disintegration, in vitro dissolution, in vitro diffusion, content uniformity, microbial analysis or a like thereof.

Further the sublingual pharmaceutical composition of the present invention remains stable by means of components of primary and secondary packaging. Preferably primary packaging in alu-alu pouch, HDPE based packaging, blisters, or bottles and secondary packaging in carton or corrugated boxes.

The pharmaceutical composition of the present invention can be used in a method for the treatment of one or more lysosomal storage diseases selected from the group comprising of, Gaucher disease, Sphingolipidoses, Farber disease, Krabbe disease, Fabry disease, Schindler disease, Tay-Sachs disease and Niemann-Pick disease.

According to another embodiment, the pharmaceutical compositions of the present invention remain stable physically and chemically which fulfils requirement of ICH and/or pharmacopoeia guidelines.

Further the pharmaceutical compositions of the present invention remain stable during its shelf-life period as per ICH and/or pharmacopoeia guidelines.

The present invention is illustrated below by reference to the following example. However, one skilled in the art will appreciate that the specific methods and results discussed are merely illustrative of the invention, and not to be construed as limiting the invention, as many variations thereof are possible without departing from the scope of the invention as described in the appended claims.

### Examples

### Example 1: Sublingual tablet of eliglustat prepared by direct compression method

**[Table 1]**

| Sr. No. | **Ingredients** | Amount %w/w |
|---|---|---|
| 1 | Eliglustat tartrate | 5-30 |
| 2 | Microcrystalline cellulose | 20-50 |
| 3 | Mannitol | 20-30 |
| 4 | Sodium starch glycolate | 5-10 |
| 5 | Crospovidone | 5-10 |
| 6 | Sucralose | 3-7 |
| 7 | Menthol | 3-7 |
| 8 | Magnesium stearate | 0.1-3 |
| 9 | Talc | 0.1-3 |
| | **Total** | **100** |

### Procedure for preparation of sublingual tablet of eliglustat by direct com- pression method:

1) In a high shear mixer using screen # 25 about half of the required quantity of microcrystalline cellulose and mannitol is added, followed by eliglustat tartrate. The remaining quantity of microcrystalline cellulose and mannitol is mixed to above blend for about 5 minutes,
2) To the blend of step 1 crospovidone, sodium starch glycolate, sucralose, menthol and talc are added using screen # 25 and mixed for 5 minutes to achieve a uniform blend,
3) The blend from step 2 is transferred to V-blender. Magnesium stearate and talc are added and blended for about 3 minutes and
4) The lubricated blend from step 3 is then compressed into tablets using tablet press.

### Example 2: Sublingual tablet of eliglustat prepared by wet granulation method

**[Table 2]**

| Sr. No. | Ingredients | Amount %w/w |
|---|---|---|
| **Wet Granulation** | | |
| 1 | Eliglustat tartrate | 5-30 |
| 2 | Mannitol | 20-30 |
| 3 | Copovidone | 1-5 |
| 4 | Crospovidone | 5-10 |
| 5 | Ethyl alcohol * | q.s. |

| **Final Blending** | | |
|---|---|---|
| 6 | Mannitol | 20-30 |
| 7 | Crospovidone | 5-10 |
| 8 | Colloidal Silicon Dioxide | 0.1-3 |
| 9 | Sucralose | 3-7 |
| 10 | Menthol | 3-7 |
| 11 | Magnesium Stearate | 0.1-3 |
| | **Total** | **100** |

| | | |
|---|---|---|
| * Removed during processing | | |

### Procedure for preparation of sublingual tablet of eliglustat by wet granulation method:

1) In a high shear mixer, required quantity of mannitol (sift using screen # 25) is added, followed by eliglustat tartrate (sift using screen # 25) and the remaining mannitol and mixed for about 5 minutes,
2) To the blend from step 1, crospovidone and copovidone (sift using screen # 25) are added and mixed for 2 minutes to achieve a uniform blend,
3) While the blend from step 2 is still mixing for additional 3 minutes, ethyl alcohol is slowly sprayed onto the powder mix until light granulation is reached,
4) The wet granules are dried in a forced air oven at 50 °C overnight and then passed through screen # 40,
5) The dried granules from step 4 are taken in a high shear mixer and the remaining mannitol, crospovidone, colloidal silicon dioxide, sucralose (sift excipients using screen # 25) and menthol are added and mixed for 5 minutes to achieve a uniform blend,
6) The blend from step 5 is transferred to V-blender and magnesium stearate (sift using screen # 30) is added and blended for about 3 minutes and
7) The lubricated blend from step 6 is then compressed into tablets using tablet press.

### Example 3: Sublingual tablet of eliglustat prepared by cryogenic freezing method

**[Table 3]**

| **Sr. No.** | **Ingredients** | **Amount %w/w** |
|---|---|---|
| 1 | Eliglustat tartrate | 5-30 |
| 2 | Gelatin | 20-50 |
| 3 | Mannitol | 40-80 |
| 4 | Crospovidone | 5-10 |
| 5 | Aspartame | 3-7 |
| 6 | Menthol | 3-7 |
| | Total | 100 |

### Procedure for preparation of sublingual tablet of eliglustat by cryogenic freezing method:

1) Mixing the eliglustat or a pharmaceutically acceptable salt thereof in a matrix comprising of partially hydrolyzed gelatin, mannitol, crospovidone, aspartame and menthol; dissolved in water and formulated into a liquid solution or suspension,
2) The liquid is precisely filled into pre-formed blisters and passed through a specially designed cryogenic freezing process to control the ultimate size,
3) The frozen units are then transferred to freeze dryers for the lyophilisation and
4) The blisters containing the dried units are then sealed via a heat-seal process.

### Example 4: Sublingual film of eliglustat

**[Table 4]**

| **Ingredients** | **A** | **B** | **C** |
|---|---|---|---|
| | **Amount %w/w** | | |
| Eliglustat tartrate | 1-30 | 1-30 | 1-30 |
| Pullulan | 30-60 | NA | 30-60 |
| Hydroxypropylmethyl cellulose | NA | 30-60 | NA |
| Glycerin | NA | 15-40 | NA |
| PEG | 15-40 | NA | 15-40 |
| Citric acid | 7-20 | 7-20 | 7-20 |
| Aspartame | 1-8 | 1-8 | 1-8 |
| Sucralose | 2-10 | 2-10 | 2-10 |
| Menthol | 0.01-0.1 | 0.01-0.1 | 0.01-0.1 |
| Distilled water | 10 ml | 10 ml | 10 ml |

### Procedure for preparation of sublingual film of eliglustat:

1) An aqueous solution of the polymers is prepared in distilled water,
2) Eliglustat tartrate is added to the aqueous polymeric solution,
3) Plasticizer is added to above solution,
4) Sweetening agent, saliva stimulating agent and flavoring agent are also added to the above solution and
5) The solution is casted on a petri dish and dried at room temperature for 24 to 48 hrs.

## Claims

1. A transmucosal pharmaceutical composition comprising eliglustat or a pharmaceutically acceptable salt thereof and a one or more pharmaceutically acceptable excipients;
wherein the eliglustat or a pharmaceutically acceptable salt thereof is present in an amount from about 1mg to about 30mg; and
wherein the transmucosal pharmaceutical composition is for use in a method comprising administration through the mucosal lining of sublingual or buccal in oral cavity.

2. The pharmaceutical composition for use according to claim 1, wherein the pharmaceutical composition is present in the form of tablets, granules, pellets, lozenges, wafers, spray, drops, effervescent, sublingual or buccal tablets, sublingual film, pastilles.

3. The pharmaceutical composition for use according to claim 1, wherein the one or more pharmaceutically acceptable excipients are selected from diluents, disintegrants, binders, polymers, lubricants, glidants, sweetening agents, flavoring agents, coating agents, taste-masking agents, solvents or mixtures thereof.

4. The pharmaceutical composition for use according to claim 2, wherein the pharmaceutical composition is present in the form of a sublingual film, and wherein one or more pharmaceutically acceptable excipients are selected from polymers, plasticizers, taste-masking agent, sweetening agents, or flavouring agents.

5. The pharmaceutical composition for use according to claim 4, wherein the polymers are selected from water-soluble, water-swellable, water-insoluble polymers, or a combination thereof, wherein the said polymers are present in an amount from about 20% to about 80% by weight of composition.

6. The pharmaceutical composition for use according to claim 4, wherein the plasticizer is selected from the group consisting of polyalkylene oxides, such as polyethylene glycols, polypropylene glycols, polyethylene-propylene glycols, organic plasticizers with low molecular weights, such as glycerol, glycerol monoacetate, diacetate or triacetate, triacetin, polysorbate, cetyl alcohol, propylene glycol, sorbitol, sodium diethylsulfosuccinate, triethyl citrate, tributyl citrate or combinations thereof, wherein the said plasticizer is present in an amount from about 0.5% to about 40% by weight of the composition.

7. The pharmaceutical composition for use according to claim 1, wherein the eliglustat or a pharmaceutically acceptable salt thereof is present in an amount from about 1% to about 80% by weight of composition.

8. The pharmaceutical composition for use according to claim 1, wherein the pharmaceutical composition disintegrates within 60 seconds or less in oral cavity.

9. The pharmaceutical composition for use according to claim 1, wherein the pharmaceutical composition comprising from about 5% to about 30% w/w of eliglustat tartrate, from about 30% to about 70% w/w of diluent, from about 2% to about 8% w/w of binder, from about 5% to about 20% w/w of disintegrant, from about 0% to about 3% w/w of lubricant, from about 0% to about 3% w/w of glidant, from about 5% w/w or less of sweetening agent, from about 5% w/w or less of flavoring agent, from about 0% to about 10% w/w of taste-masking agents, from about 3% w/w or less of coloring agent and optionally from about 1% to about 5% w/w of film coating substance.

10. The pharmaceutical composition for use according to claim 1, wherein the pharmaceutical composition is prepared by a process including dry granulation, wet granulation, direct compression, roller compaction, fluidized bed granulation, solid-dispersion, rapid mixture granulation, solvent evaporation, hot-melt extrusion, freeze-drying, lyophilisation.

11. The pharmaceutical composition for use according to claim 1, wherein the pharmaceutical composition comprises sublingual film of eliglustat or a pharmaceutically acceptable salt thereof, wherein the sublingual film is prepared by the process comprising the steps of:
a) preparing an aqueous solution of the polymers in distilled water,
b) adding eliglustat or a pharmaceutically acceptable salt thereof into the aqueous polymeric solution of step a),
c) adding plasticizer to the solution of step b),
d) adding sweetening agent, saliva stimulating agent and flavoring agent to the solution of step c), and
e) drying the solution at room temperature for 24 to 48 hrs.

12. The pharmaceutical composition for use according to claims 1, for use in the treatment of lysosomal storage diseases, wherein the said lysosomal storage diseases selected from the group consisting of, Gaucher disease, Sphingolipidoses, Farber disease, Krabbe disease, Fabry disease, Schindler disease, Tay-Sachs disease and Niemann-Pick disease.

## Patentansprüche

1. Transmukosale pharmazeutische Zusammensetzung, umfassend Eliglustat oder ein pharmazeutisch verträgliches Salz davon und einen oder mehrere pharmazeutisch verträgliche Hilfsstoffe; wobei das Eliglustat oder ein pharmazeutisch verträgliches Salz davon in einer Menge von etwa 1 mg bis etwa 30 mg vorliegt; und wobei die transmukosale pharmazeutische Zusammensetzung zur Verwendung in einem Verfahren bestimmt ist, umfassend eine Verabreichung durch die Schleimhautauskleidung sublingual oder bukkal in einer Mundhöhle.

2. Pharmazeutische Zusammensetzung zur Verwendung nach Anspruch 1, wobei die pharmazeutische Zusammensetzung in Form von Tabletten, Granulat, Pellets, Lutschtabletten, Oblaten, Spray, Tropfen, Brause-, sublingualen oder bukkalen Tabletten, sublingualem Film, Pastillen vorliegt.

3. Pharmazeutische Zusammensetzung zur Verwendung nach Anspruch 1, wobei der eine oder die mehreren pharmazeutisch verträglichen Hilfsstoffe ausgewählt sind aus Verdünnungsmitteln, Sprengmitteln, Bindemitteln, Polymeren, Gleitmitteln, Fließregulierungsmitteln, Süßungsmitteln, Aromastoffen, Beschichtungsmitteln, Geschmacksmaskierungsmitteln, Lösungsmitteln oder Mischungen davon.

4. Pharmazeutische Zusammensetzung zur Verwendung nach Anspruch 2, wobei die pharmazeutische Zusammensetzung in Form eines sublingualen Films vorliegt und wobei ein oder mehrere pharmazeutisch verträgliche Hilfsstoffe ausgewählt sind aus Polymeren, Weichmachern, Geschmacksmaskierungsmitteln, Süßungsmitteln oder Aromastoffen.

5. Pharmazeutische Zusammensetzung zur Verwendung nach Anspruch 4, wobei die Polymere ausgewählt sind aus wasserlöslichen, wasserquellbaren, wasserunlöslichen Polymeren oder einer Kombination davon, wobei die Polymere in einer Menge von etwa 20 bis etwa 80 Gew.-% der Zusammensetzung vorliegen.

6. Pharmazeutische Zusammensetzung zur Verwendung nach Anspruch 4, wobei der Weichmacher ausgewählt ist aus der Gruppe, bestehend aus Polyalkylenoxiden, wie Polyethylenglykolen, Polypropylenglykolen, Polyethylenpropylenglykolen, niedermolekularen organischen Weichmachern, wie Glycerin, Glycerinmonoacetat, -diacetat oder -triacetat, Triacetin, Polysorbat, Cetylalkohol, Propylenglykol, Sorbitol, Natriumdiethylsulfosuccinat, Triethylcitrat, Tributylcitrat oder Kombinationen davon, wobei der Weichmacher in einer Menge von etwa 0,5 bis etwa 40 Gew.-% der Zusammensetzung vorliegt.

7. Pharmazeutische Zusammensetzung zur Verwendung nach Anspruch 1, wobei das Eliglustat oder ein pharmazeutisch verträgliches Salz davon in einer Menge von etwa 1 bis etwa 80 Gew.-% der Zusammensetzung vorliegt.

8. Pharmazeutische Zusammensetzung zur Verwendung nach Anspruch 1, wobei die pharmazeutische Zusammensetzung in der Mundhöhle innerhalb von 60 Sekunden oder weniger zerfällt.

9. Pharmazeutische Zusammensetzung zur Verwendung nach Anspruch 1, wobei die pharmazeutische Zusammensetzung etwa 5 bis etwa 30 Gew.-% Eliglustat-Tartrat, etwa 30 bis etwa 70 Gew.-% Verdünnungsmittel, etwa 2 bis etwa 8 Gew.-% Bindemittel, etwa 5 bis etwa 20 Gew.-% Sprengmittel, etwa 0 bis etwa 3 Gew.-% Gleitmittel, etwa 0 bis etwa 3 Gew.-% Fließregulierungsmittel, etwa 5 Gew.-% oder weniger Süßungsmittel, etwa 5 Gew.-% oder weniger Aromastoff, etwa 0 bis etwa 10 Gew.-% Geschmacksmaskierungsmittel, etwa 3 Gew.-% oder weniger Farbstoff und gegebenenfalls etwa 1 bis etwa 5 Gew.-% Filmbeschichtungssubstanz umfasst.

10. Pharmazeutische Zusammensetzung zur Verwendung nach Anspruch 1, wobei die pharmazeutische Zusammensetzung durch ein Verfahren hergestellt wird, das Trockengranulierung, Nassgranulierung, direktes Pressen, Walzenverdichtung, Wirbelschichtgranulierung, Feststoffdispersion, Schnellmischungsgranulierung, Lösungsmittelverdampfung, Heißschmelzextrusion, Gefriertrocknung, Lyophilisierung beinhaltet.

11. Pharmazeutische Zusammensetzung zur Verwendung nach Anspruch 1, wobei die pharmazeutische Zusammensetzung einen sublingualen Film von Eliglustat oder einem pharmazeutisch verträglichen Salz davon umfasst, wobei der sublinguale Film durch das Verfahren hergestellt wird, umfassend die folgenden Schritte:
a) Herstellen einer wässrigen Lösung der Polymere in destilliertem Wasser,
b) Zugeben von Eliglustat oder einem pharmazeutisch verträglichen Salz davon zu der wässrigen polymeren Lösung aus Schritt a),
c) Zugeben eines Weichmachers zu der Lösung aus Schritt b),
d) Zugeben eines Süßungsmittels, eines speichelstimulierenden Mittels und eines Aromastoffs zu der Lösung aus Schritt c) und
e) Trocknen der Lösung bei Raumtemperatur über 24 bis 48 Stunden.

12. Pharmazeutische Zusammensetzung zur Verwendung nach Anspruch 1 zur Verwendung bei der Behandlung von lysosomalen Speicherkrankheiten, wobei die lysosomalen Speicherkrankheiten ausgewählt sind aus der Gruppe, bestehend aus Morbus Gaucher, Sphingolipidosen, Morbus Farber, Morbus Krabbe, Morbus Fabry, α-N-Acetylgalactosaminidase-Mangel, Morbus Tay Sachs und Morbus Niemann-Pick.

## Revendications

1. Composition pharmaceutique transmucosale comprenant de l'éliglustat ou un sel pharmaceutiquement acceptable de celui-ci et un ou plusieurs excipients pharmaceutiquement acceptables ;
dans laquelle l'éliglustat ou un sel pharmaceutiquement acceptable de celui-ci est présent en une quantité d'environ 1 mg à environ 30 mg ; et
dans laquelle la composition pharmaceutique transmucosale est pour une utilisation dans un procédé comprenant l'administration à travers la muqueuse sublinguale ou buccale dans la cavité buccale.

2. Composition pharmaceutique pour une utilisation selon la revendication 1, dans laquelle la composition pharmaceutique est présente sous forme de comprimés, de granulés, de pellets, de comprimés à sucer, de plaquettes, de spray, de gouttes, de comprimés effervescents, sublinguaux ou buccaux, de film sublingual, de pastilles.

3. Composition pharmaceutique pour une utilisation selon la revendication 1, dans laquelle les un ou plusieurs excipients pharmaceutiquement acceptables sont choisis parmi des diluants, des délitants, des liants, des polymères, des lubrifiants, des glissants, des agents édulcorants, des agents aromatisants, des agents d'enrobage, des agents de masquage de goût, des solvants ou des mélanges de ceux-ci.

4. Composition pharmaceutique pour une utilisation selon la revendication 2, dans laquelle la composition pharmaceutique est présente sous la forme d'un film sublingual, et dans laquelle un ou plusieurs excipients pharmaceutiquement acceptables sont choisis parmi des polymères, des plastifiants, un agent de masquage de goût, des agents édulcorants ou des agents aromatisants.

5. Composition pharmaceutique pour une utilisation selon la revendication 4, dans laquelle les polymères sont choisis parmi des polymères hydrosolubles, gonflables dans l'eau, insolubles dans l'eau, ou une combinaison de ceux-ci, dans laquelle lesdits polymères sont présents en une quantité d'environ 20 % à environ 80 % en poids de la composition.

6. Composition pharmaceutique pour une utilisation selon la revendication 4, dans laquelle le plastifiant est choisi dans un groupe comprenant les oxydes de polyalkylène, tels que les polyéthylène glycols, les polypropylène glycols, les polyéthylène-propylène glycols, les plastifiants organiques à faible poids moléculaire, tels que le glycérol, le monoacétate, le diacétate ou le triacétate de glycérol, la triacétine, le polysorbate, l'alcool cétylique, le propylène glycol, le sorbitol, le diéthylsulfosuccinate de sodium, le citrate de triéthyle, le citrate de tributyle ou des combinaisons de ceux-ci, dans laquelle ledit plastifiant est présent en une quantité d'environ 0,5 % à environ 40 % en poids de la composition.

7. Composition pharmaceutique pour une utilisation selon la revendication 1, dans laquelle l'éliglustat ou un sel pharmaceutiquement acceptable de celui-ci est présent en une quantité d'environ 1 % à environ 80 % en poids de la composition.

8. Composition pharmaceutique pour une utilisation selon la revendication 1, dans laquelle la composition pharmaceutique se désintègre en 60 secondes ou moins dans la cavité buccale.

9. Composition pharmaceutique pour une utilisation selon la revendication 1, dans laquelle la composition pharmaceutique comprend d'environ 5 % à environ 30 % p/p de tartrate d'éliglustat, d'environ 30 % à environ 70 % p/p de diluant, d'environ 2 % à environ 8 % p/p de liant, d'environ 5 % à environ 20 % p/p de délitant, d'environ 0 % à environ 3 % p/p de lubrifiant, d'environ 0 % à environ 3 % p/p de glissant, d'environ 5 % p/p ou moins d'agent édulcorant, d'environ 5 % p/p ou moins d'agent aromatisant, d'environ 0 % à environ 10 % p/p d'agents de masquage de goût, d'environ 3 % p/p ou moins d'agent colorant et éventuellement d'environ 1 % à environ 5 % p/p de substance d'enrobage.

10. Composition pharmaceutique pour une utilisation selon la revendication 1, dans laquelle la composition pharmaceutique est préparée par un processus comportant la granulation sèche, la granulation humide, la compression directe, le compactage au rouleau, la granulation en lit fluidisé, la dispersion solide, la granulation rapide de mélange, l'évaporation de solvant, l'extrusion à chaud, la cryodessication, la lyophilisation.

11. Composition pharmaceutique pour une utilisation selon la revendication 1, dans laquelle la composition pharmaceutique comprend un film sublingual d'éliglustat ou un sel pharmaceutiquement acceptable de celui-ci, dans laquelle le film sublingual est préparé par le processus comprenant les étapes suivantes :
a) préparation d'une solution aqueuse des polymères dans de l'eau distillée,
b) ajout d'éliglustat ou d'un sel pharmaceutiquement acceptable de celui-ci dans la solution polymère aqueuse de l'étape a),
c) ajout de plastifiant à la solution de l'étape b),
d) ajout d'un agent édulcorant, d'un agent stimulant la salive et d'un agent aromatisant à la solution de l'étape c), et
e) séchage de la solution à température ambiante pendant 24 à 48 heures.

12. Composition pharmaceutique pour une utilisation selon la revendication 1, pour une utilisation dans le traitement des maladies de stockage lysosomal, dans laquelle lesdites maladies de stockage lysosomal sont choisies dans un groupe comprenant la maladie de Gaucher, les sphingolipidoses, la maladie de Farber, la maladie de Krabbe, la maladie de Fabry, la maladie de Schindler, la maladie de Tay-Sachs et la maladie de Niemann-Pick.
